Europäisches Patentamt

European Patent Office

Office européen des brevets

(19)

(11) Publication number: **0 360 361**
**A1**

(12) **EUROPEAN PATENT APPLICATION**

(21) Application number: 89202413.4

(22) Date of filing: 25.09.89

(51) Int. Cl.⁵: **C12N 1/11 , C12N 5/10 ,**
**C12N 1/19 , C12N 15/53 ,**
**C12P 33/00 , A61K 31/575**

(30) Priority: 23.09.88 EP 88202080

(43) Date of publication of application:
28.03.90 Bulletin 90/13

(84) Designated Contracting States:
AT BE CH DE ES FR GB GR IT LI LU NL SE

(71) Applicant: **GIST-BROCADES N.V.**
**Wateringseweg 1**
**NL-2611 XT Delft(NL)**

(72) Inventor: **Slijkhuis, Harmen**
**Zuidersingel 27**
**NL-2651 AE Berkel en Rodenrijs(NL)**
Inventor: **Selten, Gerardus Cornelis Maria**
**Sterrenweg 81**
**NL-2651 HZ Berkel en Rodenrijs(NL)**
Inventor: **Smaal, Eric Bastiaan**
**Doelenstraat 93**
**NL-2611 NS Delft(NL)**

(54) **Process for the multiple oxidation of steroids and genetically engineered cells to be used therefor.**

(57) Genetically engineered host cells are provided which are able to carry out simultaneously oxidations of steroids, preferably the simultaneous introduction of the 17α-hydroxyl and the $C_{21}$-hydroxyl group. In particular the oxidation is carried out with cells into which DNA has been introduced which encodes for at least two proteins involved in the biological pathway of cholesterol to hydrocortisone. Suited host cells comprise species of Bacillus, Saccharomyces or Kluyveromyces. The new host cells are suited for biochemical oxidations of cholesterol, pregnenolone, progesterone and 17α-hydroxy-progesterone, which are intermediates in said biological pathway.

The host cells are also useful for the ultimate preparation of a multigenic system for the one-step conversion of cholesterol into hydrocortisone.

EP 0 360 361 A1

# PROCESS FOR THE MULTIPLE OXIDATION OF STEROIDS AND GENETICALLY ENGINEERED CELLS TO BE USED THEREFOR

The invention relates to a multiple oxidation process for the preparation of pharmaceutically useful steroids.

## BACKGROUND OF THE INVENTION

11β,17α,21-Trihydroxy-4-pregnene-3,20-dione (hydrocortisone) is an important pharmaceutical steroid, used for its pharmacological properties as a corticosteroid and as a starting compound for the preparation of numerous useful steroids, particularly other corticosteroids. Hydrocortisone is produced in the adrenal cortex of vertebrates and was originally obtained, in small amounts only, by a laborious extraction from adrenal cortex tissue. Only after structure elucidation new production routes were developed, characterised by a combination of chemical synthesis steps and microbiological conversions. Only because the starting compounds which are employed such as sterols, bile acids and sapogenins are abundant and cheap, the present processes afford a less expensive product, but these still are rather complicate. Several possibilities were envisaged to improve the present processes, and also biochemical approaches have been tried.

One attempt was to have a suited starting steroid converted in an in vitro biochemical system using the isolated adrenal cortex proteins which are known to be responsible for the enzymatical conversion in vivo of steroids to hydrocortisone. However, the difficult isolation of the proteins and the high price of the necessary cofactors, appeared to be prohibitive for an economically attractive large scale process. Another approach was to keep the catalysing proteins in their natural environment and to have the adrenal cortex cells produce in a cell culture the desired hydrocortisone. But due to the low productivity of the cells in practice it appeared to be impossible to make such a biochemical process economically attractive.

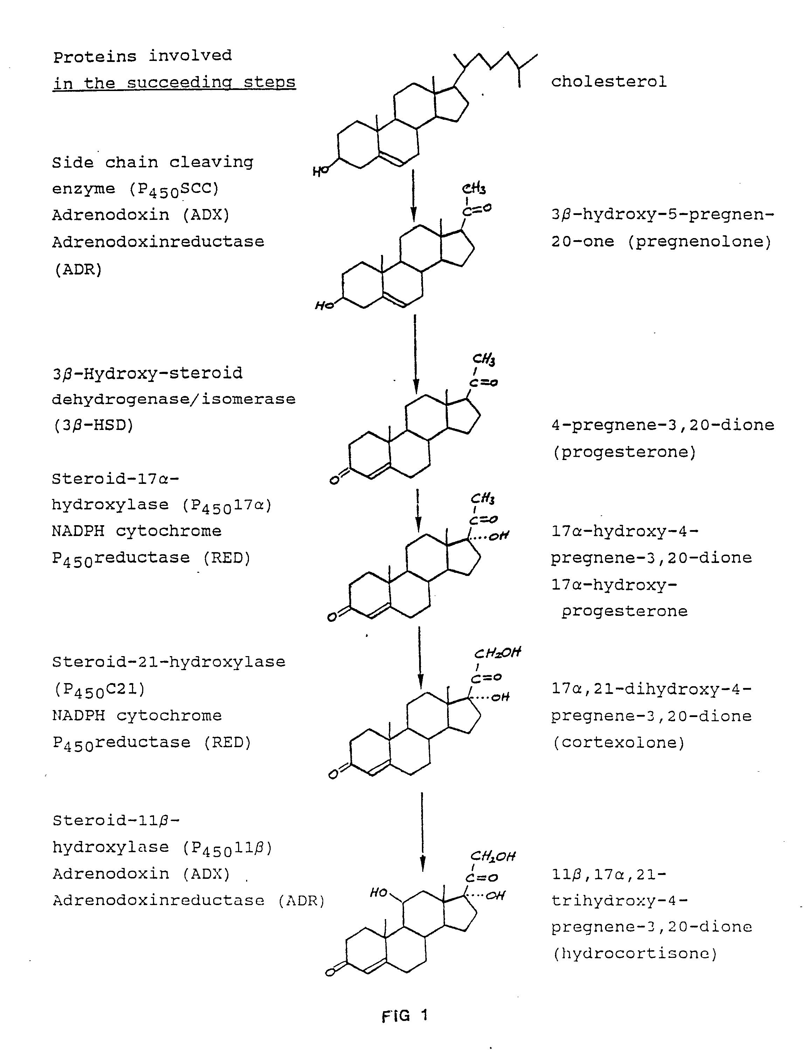

The in vivo process in the adrenal cortex of mammals and other vertebrates constitutes a biochemical pathway, which starts with cholesterol and via various intermediate compounds eventually affords hydrocortisone (see figure 1). Eight proteins are directly involved in this pathway, five of them being enzymes, among which four cytochrome $P_{450}$ enzymes, and the other three being electron transferring proteins.

The first step is the conversion of cholesterol to 3β-hydroxy-5-pregnen-20-one (pregnenolone). In this conversion, a mono-oxygenase reaction, three proteins are involved: side chain cleaving enzyme ($P_{450}$SCC, a heme-Fe-containing protein), adrenodoxin (ADX, a $Fe_2S_2$ containing protein) and adrenodoxinreductase (ADR, a FAD-containing protein). Besides cholesterol as a substrate the reaction further requires molecular oxygen and NADPH.

Subsequently pregnenolone is converted by dehydrogenation/isomerisation to 4-pregnene-3,20-dione (progesterone).

This reaction, catalysed by the protein 3β-hydroxysteroid dehydrogenase/isomerase (3β-HSD), requires pregnenolone and $NAD^+$.

To obtain hydrocortisone progesterone subsequently is hydroxylated at three positions which conversions are catalysed by mono-oxygenases. In the conversion of progesterone into 17α-hydroxyprogesterone two proteins are involved:

steroid 17α-hydroxylase ($P_{450}$17α, a heme-Fe-containing protein) and NADPH cytochrome $P_{450}$reductase (RED, a FAD- and FMN-containing protein). The reaction consumes progesterone, molecular oxygen and NADPH.

For the conversion of 17α-hydroxyprogesterone into 17α,21-dihydroxy-4-pregnene-3,20-dione (cortexolone), also two proteins are needed: steroid-21-hydroxylase ($P_{450}$C21, a heme-Fe-containing protein) and the before-mentioned protein RED. The reaction consumes 17α-hydroxyprogesterone, molecular oxygen and NADPH.

In the conversion of cortexolone into hydrocortisone, three proteins are involved: steroid 11β-hydroxylase ($P_{450}$11β), a heme-Fe-containing protein, and the above-mentioned proteins ADX and ADR.

As described above cytochrome $P_{450}$ proteins are enzymes which are essential for the biochemical conversion of cholesterol to hydrocortisone. These enzymes belong to a larger group of cytochrome $P_{450}$ proteins (or shortly $P_{450}$ proteins). They have been encountered in prokaryotes (various bacteria) and eukaryotes (yeasts, moulds, plants and animals). In mammals high levels of $P_{450}$ proteins are found in the adrenal cortex, ovary, testes and liver.

Many of these proteins have been purified and are well characterized now. Their specific activity has been determined. Recently a number of reviews on this subject have been published, such as K. Ruckpaul and H. Rein (eds), "Cytochrome P450" and P.R. Ortiz de Montellano (ed.) "Cytochrome P450, structure, mechanism and biochemistry". Cytochrome P450 proteins are characterized by their specific absorbance maximum at 450 nm after reduction with carbon monoxide. In prokaryotic organisms the P450 proteins are either membrane bound or cytoplasmatic. As far as the bacterial P450 proteins have been studied in detail (e.g. P450meg and P450cam) it has been shown that a ferredoxin and a ferredoxinreductase are involved in the hydroxylating activity. For eukaryotic organisms, two types of P450 proteins, I and II have been described. Their two differences reside in:

1. subcellular localisation, type I is localized in the microsomal fraction and type II is localized in the inner membrane of mitochondria;

2. the way the electrons are transferred to the P450 protein. Type I is reduced by NADPH via a P450reductase, whereas type II is reduced by NADPH via a ferredoxin-reductase (e.g. adrenodoxinreductase) and a ferredoxin (e.g. adrenodoxin).

According to EP-A-0281245 cytochrome P450 enzymes can be prepared from Streptomyces species and used for the hydroxylation of chemical compounds.

The enzymes are used in isolated form, which is a rather tedious and expensive procedure.

JP-A-62236485 (Derwent 87-331234) teaches that it is possible to introduce into Saccharomyces cerevisiae the genes of liver cytochrome P450 enzymes and to express them affording enzymes which may be used for their oxidation activity.

However, in the above references there is no indication to the use of cytochrome P450 enzymes for the preparation of steroid compounds.

According to our copending application EP-A-89201173.5, filed on May 8, 1989, entitled: "Process for the biochemical oxidation of steroids and genetically engineered cells to be used therefor", incorporated herein by reference, a multiplicity of expression cassettes is provided for production of proteins necessary in the construction of a multigenic system for the oxidative conversion of inexpensive steroid starting materials to more rare and expensive end products, wherein such conversion is carried out in native systems through a multiplicity of enzyme-catalyzed and cofactor-mediated conversions.

According to that application expression cassettes are provided effective in a recombinant host cell to express a heterologous coding DNA sequence, wherein said coding sequence encodes an enzyme which is able, alone or in cooperation with additional protein, to catalyze a separate oxidation step in the biological pathway for the conversion of cholesterol to hydrocortisone. The expression cassettes therefore, include those sequences capable of producing, in a recombinant host, the following proteins: side-chain cleaving enzyme (P450SCC); adrenodoxin (ADX); adrenodoxin reductase (ADR); 3$\beta$-hydroxy-steroid dehydrogenase/isomerase (3$\beta$-HSD); steroid 17$\alpha$-hydroxylase (P450 17$\alpha$); NADPH cytochrome P450 reductase (RED); steroid-21-hydroxylase (P450 C21); and steroid 11$\beta$-hydroxylase (P450 11$\beta$).

Said application further discloses recombinant host cells transformed with these vectors or with the expression cassettes of the invention, to methods to produce the above enzymes and to use these enzymes for oxidation, to processes to use said host cells for specific oxidations in a culture broth and to pharmaceutical compositions containing compounds prepared by said processes.

In particular that application deals with the preparation and culturing of cells which are suited to be employed in large scale biochemical production reactors and the use of these cells for the oxidation of compounds and particularly for the production of steroids, shown in figure 1. Each of the depicted reactions can be carried out separately. Interchange of steps in a multi-step reaction is included. Micro-organisms are preferred hosts but other cells may be used as well, such as cells of plants or animals, optionally applied in a cell culture or in the tissue of living transgenic plants or animals.

Those cells are obtained by the genetical transformation of suitable receptor cells, preferably cells of suited microorganisms, with vectors containing DNA sequences encoding the proteins involved in the conversion of cholesterol to hydrocortisone, comprising side-chain cleaving enzyme (P450SCC), adrenodoxin (ADX), adrenodoxin reductase (ADR), 3$\beta$-hydroxy-steroid dehydro-genase/isomerase (3$\beta$-HSD), steroid-17$\alpha$-hydroxylase (P450 17$\alpha$), NADPH cytochrome P450 reductase (RED), steroid-21 hydroxylase (P450 C21) and steroid-11$\beta$-hydroxylase (P450 11$\beta$). Some host cells may already produce of their own one or more of the necessary proteins at a sufficient level and therefore have to be transformed with the supplementary DNA sequences only. Such possibly own proteins are ferredoxin, ferredoxin reductase, P450-reductase, and 3$\beta$-hydroxy-steroid dehydrogenase/isomerase.

For retrieval of the sequences which encode proteins which are involved in the conversion of cholesterol to hydrocortisone suitable DNA sources have been selected.

An appropriate source for the retrieval of DNA encoding all proteins involved in the conversion of cholesterol to hydrocortisone is the adrenal cortex tissue of vertebrates e.g. bovine adrenal cortex tissue.

3

Also from various micro-organisms the relevant DNA can be retrieved, e.g. from Pseudomonas testosteroni, Streptomyces griseocarneus or Brevibacterium sterolicum for DNA encoding the 3$\beta$-hydroxy-steroid dehydrogenase/isomerase and from Curvularia lunata or Cunninghamella blakesleeana for DNA encoding proteins involved in the 11$\beta$-hydroxylation of cortexolone. The DNA-sequences coding for the proteins bovine $P_{450}$SCC, bovine $P_{450}11\beta$ or a microbial equivalent protein, bovine adreno-doxin, bovine adrenodoxin reductase, 3$\beta$-hydroxy-steroid dehydrogenase/isomerase of bovine or microbial origin, bovine $P_{450}17\alpha$, bovine $P_{450}C21$ and NADPH cytochrome $P_{450}$ reductase of bovine or microbial origin, were isolated according to the following steps:

### 1. Eukaryotic sequences (cDNA's)

a. Total RNA was prepared from appropriate tissue

b. PolyA$^+$ containing RNA was transcribed into double stranded cDNA and ligated into bacteriophage vectors

c. The obtained cDNA library was screened with $^{32}$P-labeled oligomers specific for the desired cDNA or by screening an isopropyl-$\beta$-D-thiogalactopyranoside (IPTG)-induced lambda-gt11 cDNA library using a specific ($^{125}$I-labeled) antibody

d. cDNA inserts of positive plaque forming units (pfu's) were inserted into appropriate vectors to verify:
- the entire length of the cDNA by nucleotide sequencing

### 2. Prokaryotic genes

a. Genomic DNA was prepared from an appropriate micro organism

b. To obtain a DNA library DNA fragments were cloned into appropriate vectors and transformed to an appropriate E.Coli host

c. The DNA library was screened with $^{32}$P-labeled oligomers specific for the gene of interest or by screening an IPTG-induced lambda-gt11 DNA library using a specific ($^{125}$I-labeled) antibody

d. Plasmids of positive colonies were isolated and inserted DNA fragments subcloned into appropriate vectors to verify:
- the entire length of the gene Note: According to an improved method the particular cDNA (eukaryotic sequences) or gene (prokaryotic sequences) was amplified using two specific oligomers by the method known as the polymerase chain reaction (PCR) (Saiki et al, Science, 239, 487-491, 1988). Subsequently the amplified cDNA or DNA was inserted into the appropriate vectors.

Suitable expression cassettes are provided in which the heterologous DNA isolated according to the previous procedure, is placed between suitable control sequences for transcription and translation, which enables the DNA to be expressed in the cellular environment of a suitable host, affording the desired protein or proteins. Optionally, the initiation control sequences are followed by a secretion signal sequence.

Suitable control sequences have to be introduced together with the structural DNA by said expression cassettes. Expression is made possible by transformation of a suitable host cell with a vector containing control sequences which are compatible with the relevant host and are in operable linkage to the coding sequences of which expression is desired.

Alternatively, suitable control sequences present in the host genome are employed. Expression is made possible by transformation of a suitable host cell with a vector containing coding sequences of the desired protein flanked by host sequences enabling homologous recombination with the host genome in such a manner that host control sequences properly control the expression of the introduced DNA.

As is generally understood, the term control sequences comprises all DNA segments which are necessary for the proper regulation of the expression of the coding sequence to which they are operably linked, such as operators, enhancers and, particularly, promoters and sequences which control the translation.

The promoter which may or may not be controllable by regulating its environment. Suitable promoters for prokaryotes include, for example, the trp promoter (inducible by tryptophan deprivation), the lac promoter (inducible with the galactose analog IPTG), the $\beta$-lactamase promoter, and the phage derived $P_L$ promoter (inducible by temperature variation). Additionally, especially for expression in Bacillus, useful promoters include those for alpha-amylase, protease, Spo2, spac and O/105 and synthetic promoter sequences. A preferred promoter is the one depicted in figure 5 and denoted with "HpaII". Suitable promoters for expression in yeast include the 3-phospho-glycerate kinase promoter and those for other

4

glycolytic enzymes, as well as promoters for alcohol dehydrogenase and yeast phosphatase. Also suited are the promoters for transcription elongation factor (TEF) and lactase. Mammalian expression systems generally employ promoters derived from viruses such as the adenovirus promoters and the SV40 promoter but they also include regulatable promoters such as the metallothionein promoter, which is controlled by heavy metals or gluco-corticoid concentration. Presently viral-based insect cell expression systems are also suited, as well as expression systems based on plant cell promoters such as the nopaline synthetase promoters.

Translation control sequences include a ribosome binding site (RBS) in prokaryotic systems, whereas in eukaryotic systems translation may be controlled by a nucleotide sequence containing an initiation codon such as AUG.

In addition to the necessary promoter and the translation control sequences, a variety of other control sequences, including those regulating termination (for example, resulting in polyadenylation sequences in eukaryotic systems) may be used in controlling expression. Some systems contain enhancer elements which are desirable but mostly not obligatory in effecting expression.

According to another aspect of the above-mentioned application various vectors are described which are useful t transform the host cells. A group of vectors denoted with PGBSCC-n, where "n" is any integer from 1 to 17, is especially developed for the DNA encoding the $P_{450}SCC$ enzyme.

According to a special embodiment the pGBSCC-7 vector is adapted to enclose a further heterologous gene, coding for the ADX protein. The obtained new vector called pGBSCC/ADX-1 codes for the $P_{450}SCC$ as well as the ADX protein, both of which are produced in a functional form.

Another group of vectors denoted with pGB17α-n, where "n" is any integer from 1 to 5, is especially developed for the DNA encoding the $P_{450}17\alpha$ enzyme.

A further group of vectors denoted with pGBC21-n, where "n" is any integer from 1 to 9, is especially developed for the DNA encoding the $P_{450}C21$ enzyme.

Still another group of vectors denoted with pGB11β-n, where "n" is any integer from 1 to 4, is especially developed for the DNA encoding the $P_{450}11\beta$ enzyme.

Suitable host cells have been selected which accept the vectors of the invention and allow the introduced DNA to be expressed. When culturing the transformed host cells the proteins involved in the conversion of cholesterol to hydrocortisone appear in the cell contents. The presence of the desired DNA can be proved by DNA hybridizing procedures, their transcription by RNA hybridization, their expression by immunological assays and their activity by assessing the presence of oxidized products after incubation with the starting compound in vitro or in vivo.

Transformed microorganisms are preferred hosts, particularly bacteria (more preferably Escherichia coli and Bacillus and Streptomyces species) and yeasts (such as Saccharomyces and Kluyveromyces). Other suitable host organisms are found among plants and animals, comprising insects, of which the isolated cells are used in a cell culture, such as COS cells, $C_{127}$ cells, CHO cells, and Spodoptera frugiperda (Sfg) cells. Alternatively a transgenic plant or animal is used.

A particular type of recombinant host cells are the ones in which either two or more expression cassettes according to the invention have been introduced such as pGBSCC/ADX-1 or which have been transformed by an expression cassette coding for at least two heterologous proteins, enabling the cell to produce at least two proteins involved in the pathway of figure 1.

The prepared novel cells are not only able to produce the proteins involved in the oxidative conversion of steroids resulting eventually into hydrocortisone, but also to use these proteins on the spot in the desired oxidative conversion of the corresponding substrate compound added to the culture liquid. Steroids are preferred substrates. The cells transformed with the heterologous DNA are especially suited to be cultured with the steroids mentioned in figure 1, including other sterols such as β-sitosterol. As a result oxidized steroids are obtained.

Depending on the presence in the host cell of a multiplicity of heterologous DNA encoding proteins involved in the pathway of figure 1, several biochemical conversions are possible comprising the side-chain cleaving of a sterol and/or oxidative modifications on C11, C17, C3 and C21. Therefore the expression cassettes according to the invention are useful in constructing a multigenic system which can simultaneously effect successive intra-cellular transformations of the multiple steps in the sequence as depicted in figure 1. It may be necessary to introduce into the desired host expression cassettes, which encode in their entirety the required proteins. In some instances, one or more of the proteins involved in the pathway may already be present in the host as a natural protein exerting the same activity. For example, ferredoxin, ferredoxin reductase and $P_{450}$ reductase may already be present in the host. Under those circumstances, only the remaining enzymes must be provided by recombinant transformation.

Until recently one has not succeeded to prepare a multigenic system enabling in one biochemical process at least two steps of the biochemical pathway of figure 1.


## THE INVENTION


According to the present invention it has now been accomplished to clone in one host organism the genes which code for the proteins which are able to catalyze two separate oxidations on the steroid molecule and particularly for the proteins shown in figure 1. In particular it has been realized to clone the proteins responsible for the steroid 17α-hydroxylation and for the steroid C21-hydroxylation in one and the same host organism and to have said host organism express said proteins in a functional form. Moreover according to another aspect of the invention a process is provided in which said transformed microorganisms when grown in a fermentation medium oxidize a steroid substrate present in the medium simultaneously at two different positions of the steroid molecule. In particular a one-step process is achieved for the introduction of the ·17α- as well as the 21-hydroxylgroup.

A preferred host organism is Kluyveromyces lactis, but other host organisms and in particular microorganisms, especially those previously mentioned, can be used. More particularly the microorganisms are suitable which has been described in [country and name of our previous application] for cloning and expressing the genes of the biochemical pathway as shown in figure 1.

One way to prepare a host able to carry out a multiple steroid oxidation is to transform the host with two or more vectors each containing the gene for one oxidation step.

Another way is to have the host transformed by one vector containing an expression cassette with all genes coding for the proteins necessary for the desired multiple oxidation reaction.

According to the present invention the expression cassette contains at least two structural genes each flanked by proper control sequences. One exemplified expression cassette contains DNA encoding the proteins P450-17-alpha and P450-C21 (pGB17-alpha/C21-1).

Using the method of the invention it is possible, using methods known in the art, to prepare analogous expression cassettes and host cells containing them, with which it is possible to carry out other multiple steroid oxidations and eventually the conversion of cholesterol into hydrocortisone in a single fermentation process.


## DESCRIPTION OF THE FIGURES


Abbreviations used in all figures: $R_1$, EcoRI; H, HindIII; K, KpnI; X, XbaI; $R_v$, EcoRV, $S_I$, SacI; B, BamHI; $S_{II}$, SacII; Sal, SalI and Xh, XhoI.

Figure 1 shows a schematic overview of the proteins involved in the succeeding steps in the conversion of cholesterol in hydrocortisone as occurring in the adrenal cortex of mammals.

Figure 2 shows a physical map of the expression cassette pGB17α-5.

Figure 3 shows a physical map of the expression cassette pGBC21-9.

Figure 4 represents the construction of the expression cassette pGBC17α/C21-1, containing the coding sequence for $P_{450}17\alpha$ and $P_{450}C21$, both driven by the lactase promotor. The invention is further illustrated by the following examples which should, however, not be construed as a limitation of the invention


## Example 1


Construction and transformation of an expression casette encoding bovine cytochrome $P_{450}$ steroid 17α-hydroxylase and bovine cytochrome $P_{450}$ steroid C21-hydroxylase in the yeast Kluyveromyces lactis


(a) Construction of the expression cassette

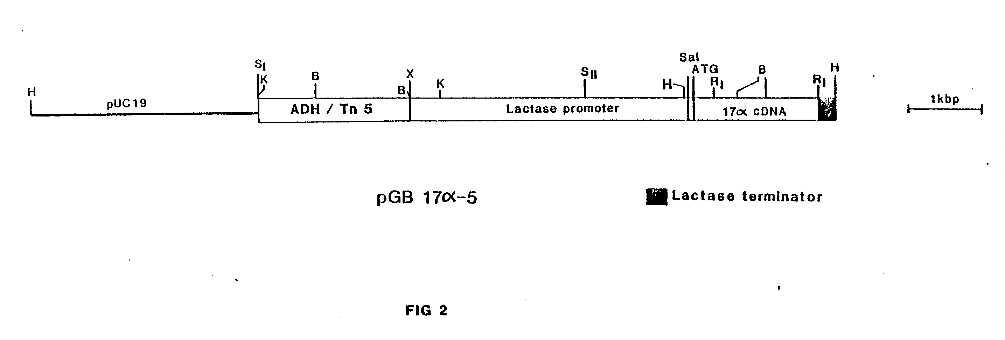

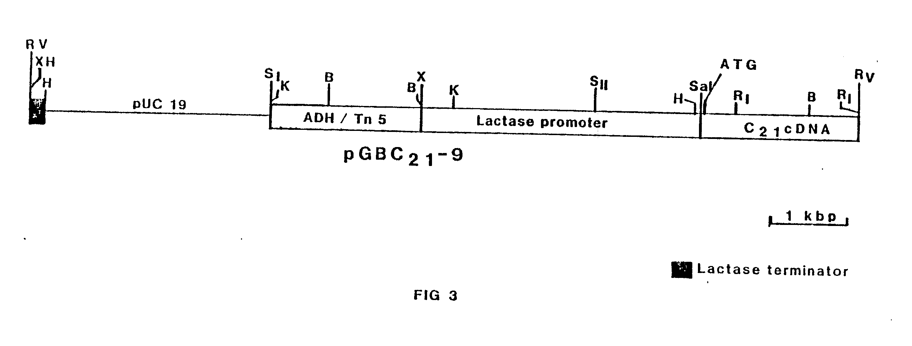

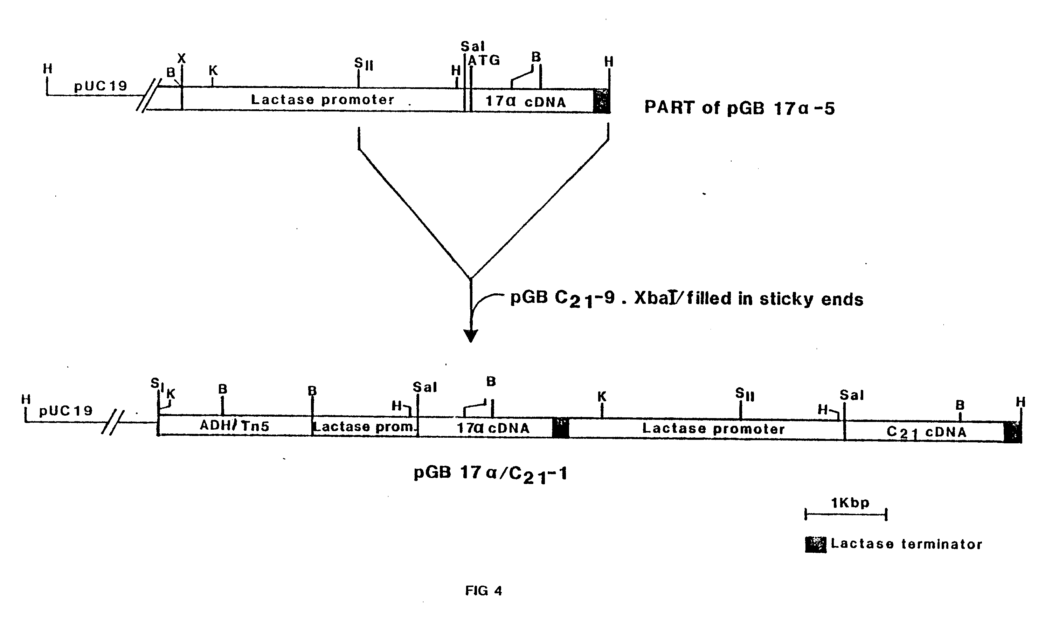

The expression cassette pGB17α-5 (figure 2) described in EP-A-89201173.5 (example 14), was digested with SacII and HindIII (partially) and sticky ends were filled in using Klenow DNA polymerase. The DNA fragment comprising a part of the lactase promoter, the sequence coding for $P_{450}17\alpha$ and the lactase terminator was separated and isolated by agarose gelelectrophoresis and subsequently inserted into pGBC21-9 (figure 3), described in EP-A-89201173.5 (example 19), which was first linearized by XbaI digestion and sticky ends filled in using Klenow DNA polymerase. The obtained expression cassette pGB17α/C21-1 (figure 4) has a unique SacII restriction site because the SacII restriction site in the lactase promotor flanking the $P_{450}17\alpha$ sequence is destroyed by the fill-in reaction.

(b) Transformation of K.lactis

Transformation of K.lactis CBS2360 was performed as described in EP-A-89201173.5, example 5(c) with 15μg pGB17α/C21-1, linearized at the unique SacII restriction site. One transformant 17α/C21-101 was further analyzed for in vitro and in vivo activity of both, $P_{450}17\alpha$ and $P_{450}C21$ (see examples 2 and 3).

Example 2

In vitro activity of $P_{450}17\alpha$ and $P_{450}C21$ obtained from Kluyveromyces lactis 17α/C21-101

K.lactis 17α/C21-101 obtained as described in example 1, K.lactis 17α-101 as described in EP-A-89201173.5 (example 14) and K.lactis CBS2360 were grown in 100 ml of medium D. Medium D contained per litre of distilled water:

| Yeast extract (Difco) | 10 g |
| Bacto Peptone (Oxoid) | 20 g |
| Dextrose | 20 g |
| pH = 6.5 | |

After sterilization and cooling to 30 °C, 25 mg of geneticin (G418 sulphate; Gibco Ltd) dissolved in 1 ml of distilled water (sterilized by membrane filtration) was added.

The cultures were grown for 72 hours at 30 °C. The cells were collected by centrifugation (4000xg, 15 minutes), the pellet washed with phosphate buffer (50 mM, pH = 7.0) and cells were collected by centrifugation (4000xg, 15 minutes). The pellet was taken up in phosphate buffer (50 mM, pH = 7.0) resulting in a suspension containing 0.5g wet weight/ml. This suspension was disrupted by sonication (Braun labsonic 1510;12x1 minute, 50 Watts). Unbroken cells were removed by centrifugation (12000xg, 15 minutes).

Cell-free extracts were assayed for $P_{450}17\alpha$ activity and $P_{450}C21$ activity by determining the production of 17α,21 dihydroxyprogesterone in the presence of NADPH. The assay mixture consisted of the following solutions:

Solution A: a 50 mM potassium phosphate buffer (pH = 7.0), containing 3 mM of EDTA, 2 mM of NADPH, 50 mM of glucose-6-phosphate and 16 units/ml glucose-6-phosphate-dehydrogenase (NADPH-regenerating system).

Solution B (substrate): a micellar solution of 80μM of [4-$^{14}$C] progesterone (30 Ci/mole) in 10% (v/v) TergitolTM NP40/ethanol (1:1,v/v) in a potassium phosphate buffer (75mM, pH = 7,5).

The assay was started by mixing 75 μl of solution A with 50 μl of solution B and 125 μl of cell-free extract. The mixture was stirred gently at 30 .C. Samples (50 μl) were drawn after 60 minutes of incubation and added to a mixture of 100 μl methanol and 50 μl chloroform. Subsequently 100 μl of chloroform and 100 μl of water were added. The chloroform layer was collected by centrifugation (5000xg, 2 minutes) and the water/methanol layer was re-extracted with 100 μl of chloroform. The two chloroform layers were combined and dried. The dry residue was dissolved in 100 μl of acetonitril/H$_2$O (9:l,v/v) and samples (50 μl) were eluted with acetonitril/H$_2$O (58:42, v/v) using an HPLC column (Chrompack lichr. 10RP18, 250x4.6 mm). In the eluate the steroid substrate and products were detected by a flowscintillationcounter and a U.V.

detector. The radioactivity of the collected fractions was determined by liquidscintillationcounting.

Using this assay it was found that a cell-free extract obtained from K.lactis 17α/C21-101 produced 17α,21 dihydroxy progesterone, whereas cell-free extracts obtained from K.lactis 17α-101 and K.lactis CBS2360 did not. The main product produced by K.lactis 17α-101 appeared to be 17α hydroxy progesterone.


## Example 3


In vivo activity of $P_{450}17\alpha$ and $P_{450}C21$ in Kluyveromyces lactis 17α/C21-101

K.lactis 17α/C21-101 obtained as described in example 1 and K.lactis CBS2360 were inoculated in 25 ml of medium D. Medium D contained per litre of distilled water:

| Yeast extract (Difco) | 10 g |
| Bacto Peptone (Oxoid) | 20 g |
| Dextrose | 20 g |
| pH = 6.5 | |

After sterilization and cooling to 30 °C, 25 mg of geneticin dissolved in 1 ml of distilled water sterilized by membrane-filtration was added to 1 litre of medium D.

Subsequently 100 μl of a solution containing the substrate [4-$^{14}$C] progesterone was added to 25 ml of the completed medium. The substrate solution contained per ml 800 μg [4-$^{14}$C] progesterone (8 Ci/mole) in 10% (v/v) TergitolTM NP40/ethanol (1:1, v/v).

The cultures were grown at 30 °C in a rotary shaker (240 rpm) and samples of 2 ml taken after 0 and 68 hours were drawn. Each sample was mixed with 2 ml of methanol. After 24 hours of extraction at 4 °C the mixtures were centrifugated (4000xg, 15 minutes). From the thus obtained supernatant samples of 200 μl were eluted with acetonitril/H$_2$O (58:42, v/v) using an HPLC column (Chrompack Lichr. 10 RP18, 250x4.6 mm).

In the eluate the steroid substrate and products were detected. The radioactivity of the collected fractions was determined by liquidscintillationcounting. One of the fractions obtained from a culture of K.lactis 17α/C21-101 grown for 68 hours clearly showed the presence of 17α,21 dihydroxyprogesterone, whereas this compound was not produced in a culture of the control strain K.lactis CBS2360.


## Claims

1. A recombinant host cell and progeny thereof comprising cells of micro-organisms, plants or animals containing, besides proper control sequences, heterologous DNA coding for proteins either from the group comprising proteins which are functional, alone or in cooperation with one or more additional proteins, of catalyzing an oxidation step in the biological pathway for conversion of cholesterol into hydrocortisone, which step is selected from the group consisting of:
the conversion of cholesterol to pregnenolone;
the conversion of pregnenolone to progesterone;
the conversion of progesterone to 17α-hydroxyprogesterone;
the conversion of 17α-hydroxyprogesterone to cortexolone;
the conversion of cortexolone to hydrocortisone,
or from the group comprising the natural proteins of the biological pathway for conversion of cholesterol into hydrocortisone being
side-chain cleaving enzyme ($P_{450}SCC$);
adrenodoxin (ADX);
adrenodoxin reductase (ADR);
3β-hydroxysteroid dehydrogenase/isomerase (3β-HSD);

8.

steroid-17α-hydroxylase (P$_{450}$17α);
NADPH cytochrome P$_{450}$ reductase (RED);
steroid-21-hydroxylase (P$_{450}$C21), and
steroid-11β hydroxylase (P$_{450}$11β),
characterized by heterologous DNA encoding at least two expressable proteins which catalyze at least two separate oxidation steps.

2. A recombinant host cell according to claim 1, characterized in that the heterologous DNA coding sequences originate from bovine species.

3. A recombinant host cell according to claims 1 or 2, characterized in that the heterologous DNA encodes at least the P$_{450}$17α as well as the P$_{450}$C21 proteins.

4. A recombinant host cell and progeny thereof according to any one of claims 1-3, characterized in that the host is a microorganism.

5. A recombinant host cell and progeny thereof according to claim 4, characterized in that the host is a species of Saccharomyces, Kluyveromyces or Bacillus or is Escherichia coli.

6. A process for the preparation of a mixture of proteins by a recombinant host cell comprising culturing cells of the recombinant host in a nutrient medium under conditions enabling the proteins to be formed and to accumulate in the culture, characterized in that the recombinant host is as defined by any one of claims 1-5.

7. A process for selective multiple oxidation of a compound in vitro, which process comprises: incubating the compound to be oxidized in the presence of at least two proteins under conditions which permit said oxidation and the accumulation of the oxidized compound in the culture liquid, followed by recovering the oxidized compound, characterized in that the proteins have been produced by the process of claim 6.

8. A process for selective multiple oxidation of a compound which process comprises: culturing recombinant host cells in the presence of said compound under conditions wherein the desired oxidation occurs and the oxidized compound accumulates in the culture broth, followed by recovering the oxidized compound, characterized in that the recombinant host cells are the cells of any one of claims 1-5.

9. A process according to claims 7 or 8, characterized in that at least two oxidation steps are carried out selected from the group consisting of: cleaving the side-chain of a sterol compound, oxidation and isomerisation of the 3-hydroxy,5(6)-dehydro group to a 3-oxo,4(5)-dehydro group, introduction of a 17α-hydroxyl group, a 21-hydroxyl group or a 11β-hydroxyl group.

10. A process according to claims 7 or 8, characterized in that the oxidation results in the simultaneous introduction of a C21-hydroxyl group as well as a C17α-hydroxyl group into pregnenolone or progesterone.

11. An expression cassette, operable in a recombinant host according to any one of claims 1-5, containing at least two genes coding for the proteins as defined in claim 1.

12. An expression cassette, according to claim 11, characterized in that the heterologous DNA encodes the enzymes P450-17alpha and P450C21 and that the expression cassette is pGB17alpha/C21-1.

13. Pharmaceutical preparations containing an active compound, which has been prepared according to any one of claims 9-12.

Proteins involved
in the succeeding steps

cholesterol

Side chain cleaving
enzyme ($P_{450}$SCC)
Adrenodoxin (ADX)
Adrenodoxinreductase
(ADR)

$3\beta$-hydroxy-5-pregnen-
20-one (pregnenolone)

$3\beta$-Hydroxy-steroid
dehydrogenase/isomerase
($3\beta$-HSD)

4-pregnene-3,20-dione
(progesterone)

Steroid-17$\alpha$-
hydroxylase ($P_{450}$17$\alpha$)
NADPH cytochrome
$P_{450}$reductase (RED)

17$\alpha$-hydroxy-4-
pregnene-3,20-dione
17$\alpha$-hydroxy-
progesterone

Steroid-21-hydroxylase
($P_{450}$C21)
NADPH cytochrome
$P_{450}$reductase (RED)

17$\alpha$,21-dihydroxy-4-
pregnene-3,20-dione
(cortexolone)

Steroid-11$\beta$-
hydroxylase ($P_{450}$11$\beta$)
Adrenodoxin (ADX)
Adrenodoxinreductase (ADR)

11$\beta$,17$\alpha$,21-
trihydroxy-4-
pregnene-3,20-dione
(hydrocortisone)

FIG 1

1kbp

H
RI

B

Sal
ATG
RI

17α cDNA

H

SII

Lactase promoter

K

X
B

ADH / Tn 5

B

SI
K

pUC19

H

pGB 17α-5

■ Lactase terminator

FIG 2

FIG 3

FIG 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | PROCEEDINGS OF THE NATIONAL ACADEMY OF SCIENCES OF THE USA, vol. 84, no. 20, October 1987, pages 7193-7197, Washington, DC, US; S.C. CHUA et al.: "Cloning of cDNA encoding steroid 11beta-hydroxylase (P450c11)" * Whole article * | 1-13 | C 12 N 1/11<br>C 12 N 5/10<br>C 12 N 1/19<br>C 12 N 15/53<br>C 12 P 33/00<br>A 61 K 31/575 |
| A | US-A-4 720 454 (P.C. WHITE et al.) * Abstract * | 1-13 | |
| A | PROC. NATL. ACAD. SCI. USA, vol. 81, September 1984, pages 5628-5632, Washington, DC, US; M.E. JOHN et al.: "Identification and characterization of cDNA clones specific for cholesterol side-chain cleavage cytochrome P-450" * Whole article * | 1-13 | |
| A | BIOCHEMICAL AND BIOPHYSICAL RESEARCH COMMUNICATIONS, vol. 145, no. 3, 30th June 1987, pages 1239-1247, Academic Press, Inc.; Y. NONAKA et al.: "Molecular cloning and sequence analysis of full-length cDNA for mRNA of adrenodoxin oxidoreductase from bovine adrenal cortex" * Whole article *<br>---      -/- | 1-13 | **TECHNICAL FIELDS SEARCHED (Int. Cl.5)**<br><br>C 12 N<br>C 12 P |

The present search report has been drawn up for all claims

| Place of search | Date of completion of the search | Examiner |
|---|---|---|
| THE HAGUE | 07-12-1989 | HUBER-MACK A. |

European Patent Office

Application Number

EP 89 20 2413

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | PROC. NATL. ACAD. SCI. USA, vol. 82, September 1985, pages 5705-5709, Washington, D.C., US; T. OKAMURA et al.: "Molecular cloning and amino acid sequence of the precursor form of bovine adrenodoxin: Evidence for a previously unidentified COOH-terminal peptide" <br> * Whole article * | 1-13 | |
| A | SCIENCE, vol. 234, December 1986, pages 1258-1261, Washington, D.C., US; M.X. ZUBER et al.: "Expression of bovine 17alpha-hydroxylase cytochrome P-450 cDNA in nonsteroidogenic (COS 1) cells" <br> * Whole article * | 1-13 | |
| A | PROC. NATL. ACAD. SCI. USA, vol. 81, April 1984, pages 1986-1990, Washington, D.C., US; P.C. WHITE et al.: "Cloning and expression of cDNA encoding a bovine adrenal cytochrome P-450 specific for steroid 21-hydroxylation" <br> * Whole article * | 1-13 | TECHNICAL FIELDS SEARCHED (Int. Cl.5) |

The present search report has been drawn up for all claims

| Place of search | Date of completion of the search | Examiner |
|---|---|---|
| THE HAGUE | 07-12-1989 | HUBER-MACK A. |

EPO FORM 1503 03.82 (P0401)